# EUROPEAN PATENT SPECIFICATION

(11) **EP 2 739 346 B1**
(45) Date of publication and mention of the grant of the patent: **28.12.2016**
(21) Application number: 11746092.3
(22) Date of filing: 05.08.2011
(51) Int. Cl.: A61N 1/36, H01R 4/18, H01R 24/40

(54) **SOUND PROCESSOR INTERCONNECTS, HEADPIECE ASSEMBLIES, AND METHODS OF MAKING THE SAME**
KLANGPROZESSORVERBINDUNGEN, KOPFSTÜCKANORDNUNGEN UND VERFAHREN ZU IHRER HERSTELLUNG
INTERCONNEXIONS DE PROCESSEUR DE SON, ENSEMBLES CASQUES ET LEURS PROCÉDÉS DE FABRICATION

(43) Date of publication of application: 11.06.2014
(73) Proprietor: Advanced Bionics AG, 8712 Stäfa (CH)
(72) Inventor: VAISHYA, Manish, Valencia, CA 91381 (US); CRAWFORD, Scott, A., Castaic, CA 91384 (US)
(74) Representative: Schwan Schorer & Partner mbB
(86) International application number: PCT/US2011/046788
(87) International publication number: WO 2013/022422

(56) References cited:
- EP-A1- 0 247 649
- DE-U1- 20 205 922
- US-A- 4 360 244
- US-A1- 2004 137 790
- US-B1- 6 361 348

## Description

### BACKGROUND

### 1. Field

The present disclosure relates generally to interconnects and headpieces for use in implantable cochlear stimulation (or "ICS") systems.

### 2. Description of the Related Art

ICS systems are used to help the profoundly deaf perceive a sensation of sound by directly exciting the intact auditory nerve with controlled impulses of electrical current. Ambient sound pressure waves are picked up by an externally worn microphone and converted to electrical signals. The electrical signals, in turn, are processed by a sound processor, converted to a pulse sequence having varying pulse widths and/or amplitudes, and transmitted to an implanted receiver circuit of the ICS system. The implanted receiver circuit is connected to an implantable electrode array that has been inserted into the cochlea of the inner ear, and electrical stimulation current is applied to varying electrode combinations to create a perception of sound. A representative ICS system is disclosed in U.S. Patent No. 5,824,022, which is entitled "Cochlear Stimulation System Employing Behind-The-Ear Sound processor With Remote Control" and incorporated herein by reference in its entirety.

As alluded to above, some ICS systems include an implantable device, a sound processor unit, and a microphone that is in communication with the sound processor unit. The implantable device communicates with the sound processor unit and, to that end, some ICS systems include a headpiece that is in communication with both the sound processor unit and the implantable device. In one type of ICS system, the sound processor unit is worn behind the ear (a "BTE unit"), while other types of ICS systems have a body worn sound processor unit (or "body worn unit"). The body worn unit, which is larger and heavier than a BTE unit, is typically worn on the user's belt or carried in the user's pocket. One example of a conventional body worn unit is the Advanced Bionics Platinum Series body worn unit.

US 2004/0137790A1 relates a connector that may be connected to a coaxial cable, having a housing section and a central conductor pin. The coaxial cable has a central conductor, an outer conductor, a dielectric layer, and an outer jacket. A coupling element may be crimped to the center conductor outside the connector. Solder may be inserted into the coupling element which is connected to the central conductor pin by inserting spring arms 48-54 into the bore.

US 4,360,244 relates to a connector unit with outer and inner shell bodies, and a coaxial cable with a center conductor, and outer shield, and an exterior sheath. A contact member is crimped onto the center conductor 28, while the center conductor is within the connector unit, by inserting a crimp tool through a porthole. The pin segment of contact member mates with a central contact member, which is within shell body, and solder is used to connect the shell body to shell body.

EP 0 247 649 A1 relates to a cochlear implant system.

Headpieces may be connected to the sound processor by a interconnect that includes a coaxial cable and connectors on each end that can be detachably connected to the headpiece and sound processor. As used herein, a connector is "detachable" if it can be readily connected to and disconnected from a corresponding connector on the associated device without disassembly or destruction of either connector. Such connectors include a plug that is connected to both the center conductor and the metallic shield of the coaxial cable. Solder connects the cable center conductor to one portion of the plug and a crimp is used to connect the metallic shield to another portion of the plug.

The present inventors have determined that conventional headpiece interconnects are susceptible to improvement. For example, cable may be twisted about its axis, bent about the connector, or pulled axially during use, and such twisting, bending or pulling may cause the plug/cable connection to fail. In particular, the connectors on such headpiece interconnects tend to be relatively small and, because crimping tools are too large to be inserted into the plug, soldering must be used to connect the center conductor of the cable to each plug. There is, however, a point of high stress concentration at the solder connection and bending and/or twisting of the center conductor at the solder connection can lead to failure. With respect to failure due to pulling, the present inventors have determined that, when the cable is pulled axially, the load on the solder connection at the cable center conductor and the crimp connection at the shield may be sufficient to result in failure of the connections.

### SUMMARY

The invention relates to a headpiece interconnect as defined claim 1 and to a connecting method as defined in claim 13. The present inventions also include headpiece assemblies with a headpiece and such an interconnect.

Such headpiece interconnects, headpiece assemblies and methods are advantageous for a variety of reasons. For example, crimping the ferrule to an intact portion of the coaxial cable in addition to the crimp body distributes the load over multiple components and, accordingly, reduces the likelihood of failure when the cable is pulled.

The above described and many other features of the present inventions will become apparent as the inventions become better understood by reference to the following detailed description when considered in conjunction with the accompanying drawings.

### BRIEF DESCRIPTION OF THE DRAWINGS

Detailed descriptions of the exemplary embodiments will be made with reference to the accompanying drawings.
FIG. 1 is a functional block diagram of an ICS system in accordance with one embodiment of a present invention.
FIG. 2 is a perspective view of an ICS system in accordance with one embodiment of a present invention.
FIG. 3 is a side view showing the end of an exemplary coaxial cable.
FIG. 4 is a perspective view of a headpiece assembly in accordance with one embodiment of a present invention.
FIG. 5 is a perspective view of a headpiece interconnect in accordance with one embodiment of a present invention.
FIG. 6 is a section view of a connector assembly in accordance with one embodiment of a present invention.
FIG. 6A is a section view of showing the connector assembly illustrated in FIG. 6 after the crimp pin has been soldered to the plug.
FIG. 7 is an end view of the connector assembly illustrated in FIG. 6.
FIG. 8 is a perspective view of a portion of the connector assembly illustrated in FIG. 6.
FIG. 9 is a side, cutaway view of a connector assembly in accordance with one embodiment of a present invention.
FIG. 9A is a partial section view of a portion of the connector assembly illustrated in FIG. 9.
FIG. 10 is a perspective view of a portion of the connector assembly illustrated in FIG. 9.
FIG. 11 is an exploded view of portions of a headpiece interconnect prior to assembly.
FIGS. 12-18 are side views showing steps in an exemplary method of assembling a headpiece interconnect.

### DETAILED DESCRIPTION OF THE EXEMPLARY EMBODIMENTS

The following is a detailed description of the best presently known modes of carrying out the inventions. This description is not to be taken in a limiting sense, but is made merely for the purpose of illustrating the general principles of the inventions.

The present inventions have application in a wide variety of systems that provide sound (i.e. either sound or a perception of sound) to the hearing impaired as well as others who require such systems on a situational basis. One example of such a system is an ICS system where an external sound processor communicates with a cochlear implant and, accordingly, the present inventions are discussed in the context of ICS systems. The present inventions are not, however, limited to ICS systems and may be used in combination with other systems for the hearing impaired that currently exist, or are yet to be developed.

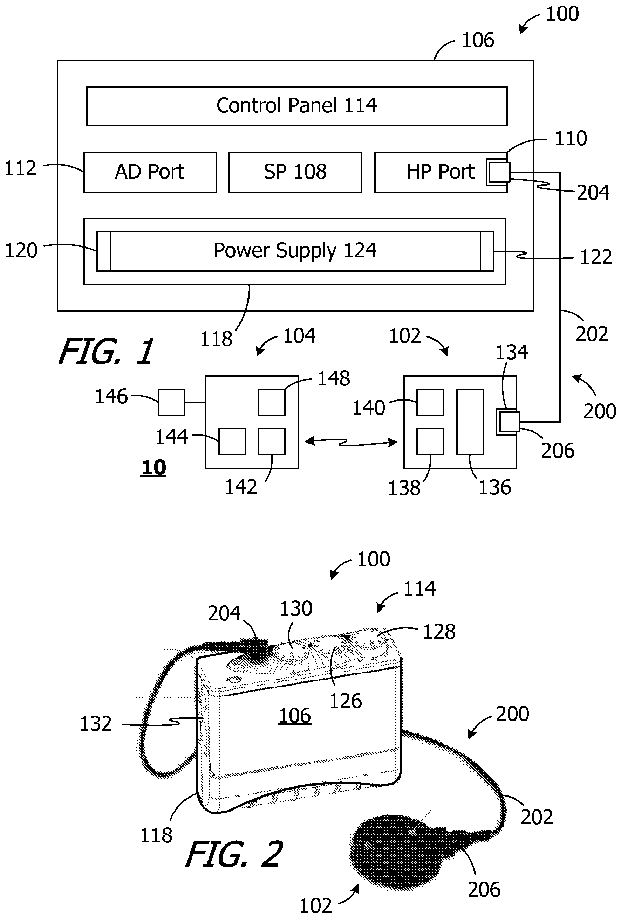

One example of a sound processor is the body worn sound processor ("sound processor") generally represented by reference numeral 100 in FIGS. 1 and 2. The exemplary sound processor 100, which may be combined with a headpiece 102 and a cochlear implant 104 to form an ICS system 10, includes a housing 106 in which and/or on which various components are supported. Such components may include, but are not limited to, sound processor circuitry 108, a headpiece port 110, an auxiliary device port 112 for an auxiliary device such as a mobile phone or a music player, a control panel 114, and a power supply receptacle 118 with electrical contacts 120 and 122 for a removable battery or other removable power supply 124 (e.g. rechargeable and disposable batteries or other electrochemical cells). Power supply receptacles are also sometimes referred to as "battery compartments" when they are intended for use with a battery. The headpiece port 110 and auxiliary device port 112 may be connected to the sound processor circuitry 108 by way of, for example, a signal splitter/combiner (not shown) such as that found in the Platinum Signal Processor body worn unit from Advanced Bionics, LLC. In the illustrated embodiment, the control panel 114 includes a volume knob 126, a program switch 128, and a sensitivity knob 130. A release button 132 is also carried on the housing 106. The release button 132 releases the power supply receptacle 118 from the housing 106.

The headpiece 102 in the exemplary ICS system 10 includes a cable port 134, a microphone 136, an antenna 138 and a positioning magnet 140. The exemplary cochlear implant 104 includes an antenna 142, an internal processor 144, a cochlear lead 146 with an electrode array, and a positioning magnet (or magnetic material) 148. The transmitter 138 and receiver 142 communicate by way of electromagnetic induction, radio frequencies, or any other wireless communication technology. The positioning magnet 140 and positioning magnet (or magnetic material) 148 maintain the position of the headpiece antenna 138 over the cochlear implant antenna 142.

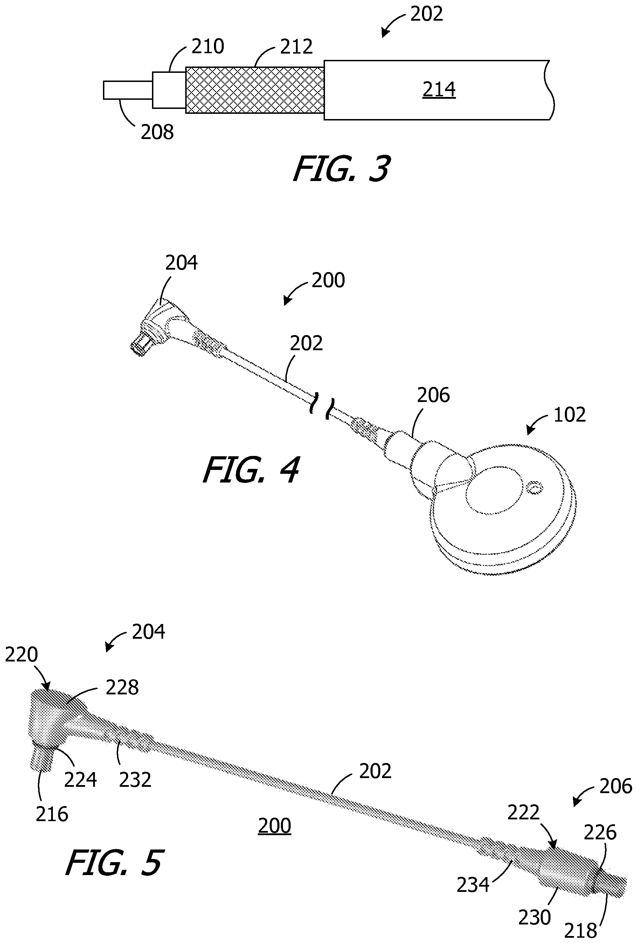

A headpiece interconnect (or "interconnect") 200 may be used to connect the headpiece port 110 on the sound processor 100 to the cable port 134 on the headpiece 102. To that end, the exemplary interconnect 200 includes a coaxial cable 202, a first connector 204 and a second connector 206. The first connector 204 and second connector 206 may be detachably connected to the corresponding connectors associated with the ports 110 and 134. Interconnects are discussed greater detail below with reference to FIGS. 3-18.

During use, the microphone 136 picks up sound from the environment and converts it into electrical impulses, and the sound processor 100 filters and manipulates the electrical impulses and sends the processed electrical signals through the cable 134 to the transmitter 138. Electrical impulses received from an auxiliary device are processed in essentially the same way. The receiver 142 receives signals from the transmitter 138 and sends the signals to the cochlear implant internal processor 144, which modifies the signals and passes them through the cochlear lead 146 to the electrode array. The electrode array may be wound through the cochlea and provides direct electrical stimulation to the auditory nerves inside the cochlea. This provides the user with sensory input that is a representation of external sound waves which were sensed by the microphone 136.

Referring to FIGS. 3-5, the coaxial cable 202 of the exemplary interconnect 200 includes a center conductor 208, a dielectric insulator 210, a metallic shield 212 (e.g., braided copper wire), and an electrically insulating jacket 214. The first and second connectors 204 and 206 include respective connector assemblies 216 and 218, which have structures that are mechanically and electrically connected to the coaxial cable center conductor 208 and metallic shield 212, and mechanically connected to outer non-conductive portions 220 and 222 and seals 224 and 226. The exemplary non-conductive portions 220 and 222 have finger grips 228 and 230, which are held by the user when the connectors 204 and 206 are attached to and detached from the associated sound processor and headpiece, as well as strain relief elements 232 and 234 that reduce the mechanical strain on the underlying portions of the coaxial cable 202 when the cable is bent relative to the connectors 204 and 206.

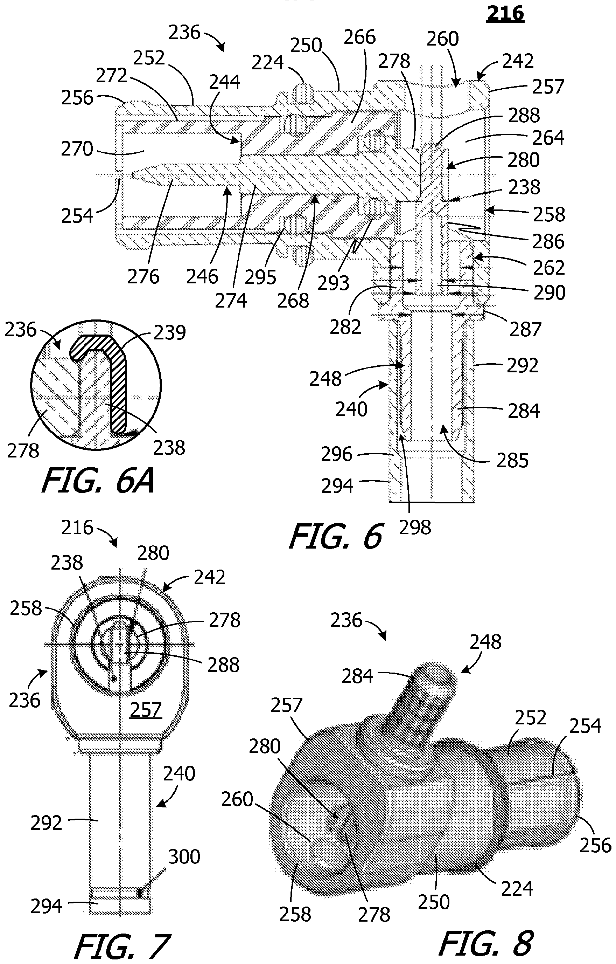

As illustrated in FIGS. 6-8, the exemplary connector assembly 216 includes a plug 236, a crimp pin 238 and a ferrule 240. During assembly, the crimp pin 238 is crimped to the center conductor 208 of the coaxial cable 202 (FIG. 3) prior to the center conductor and crimp pin being inserted into the plug 236. As used herein, the term "crimp" refers to an interconnection of at least two structures created by a deformation in at least one of the structures, as well as to the process of interconnecting at least two structures by deforming at least one of the structures. The crimp pin 238 is soldered to the plug 236 during the assembly process, as opposed to the conventional method of soldering a center conductor to a plug. The solder is represented by reference numeral 239 in FIG. 6A. Other exemplary methods of securing the crimp pin 238 to the plug 236 include staking (i.e., deforming) the end of the crimp pin, press fitting the crimp pin into the plug, providing mating threads on the crimp pin and plug, and the use of adhesives such as epoxy. The crimp pin 238 is more rigid than the cable center conductor 208. The rigidity of the crimp 238 is also such that it will not bend as the cable 202 bends and twists, and there will not be an area of stress concentration at the solder connection. The ferrule 240 is crimped over the coaxial cable 202 at two longitudinally spaced locations. Crimping the ferrule in this manner results in the pulling load being distributed over multiple components and, accordingly, reduces the likelihood that there will be failure at the solder connection.

In the exemplary implementation, the plug 236 has an outer conductor 242, an insulator 244, a center conductor 246, and a crimp body 248 that is connected to the outer conductor. The outer conductor 242 includes a base 250, an annular barrel 252 with gaps 254 and protrusions 256, and a hub 257. The gaps 254 allow the barrel 252 to flex inwardly when the plug 236 is inserted into, and the protrusions engage the inner surface of, the corresponding connector. Apertures 258, 260 and 262 extend through the hub 257 and into an open interior region 264. The aperture 258 provides access for a soldering iron during the assembly process and also, in combination with aperture 260, allows the ingress of material during the molding of the non-conductive portion 220 (FIG. 5). The aperture 262 receives a portion of the crimp body 248, as is discussed below. Suitable materials for the outer conductor 242 include, but are not limited to, beryllium copper and brass.

The exemplary insulator 244 illustrated in FIGS. 6-8 includes a main body 266 with an inner lumen 268 for the center conductor 246, an open region 270 to accommodate the socket of the corresponding connector, and an indentation 272 that allows barrel 252 of the outer conductor 242 to flex inwardly. Suitable electrically insulating materials for the insulator 244 include, but are not limited to, polytetrafluoroethylene (PTFE) and polyacetal (polyoxymethylene, or POM).

The center conductor 246 in the exemplary plug 236 is generally cylindrical in shape and includes a main portion 274, a post 276 that is inserted into the socket of the corresponding connector, and a seat 278 with slot 280 for the crimp pin 238. Suitable materials for the center conductor 246 include, but are not limited to, brass and beryllium copper.

The exemplary crimp body 248 is generally annular in shape and includes a base 282, which is located in the outer conductor aperture 262, and a support 284 onto which the coaxial cable metallic shield 212 (FIG. 3) is crimped. A lumen 285 extends through the crimp body 248, and an abutment 287 is located between the base 282 and support 284. The outer surface of the support 284 may be knurled (as shown in FIG. 8). The respective configurations of the plug 236 and the crimp body 248 are such that the connector 204 has an overall L-shape, i.e. the axis of the center conductor 246 and plug barrel 252 is perpendicular to the axis of the crimp body 248 and cable 202 (FIG. 5). Suitable materials for the crimp body 248 include, but are not limited to, brass and beryllium and copper.

The exemplary connector plug 236 is also provided with a seal 293 that is compressed between the insulator 244 and the center conductor 246 as well as a seal 295 that is compressed between the insulator and the outer conductor 242. The seals 293 and 295 protect the interior of the connector plug 236 from moisture. Suitable materials for the internal seals 293 and 295 and the external seal 224 include, but are not limited to, silicone rubber and Neoprene synthetic rubber.

The exemplary crimp pin 238 is generally cylindrical in shape and includes a main portion 286 and a narrow portion 288. The main portion 286 has a lumen 290 for the coaxial cable center conductor 208, and the narrow portion 288 is configured to fit into a slot 280 on the center conductor seat 278. The narrow portion 288 is soldered to the center conductor 246. Suitable materials for the crimp pin 238 include, but are not limited to, brass and beryllium copper.

Referring to FIGS. 6 and 7, the exemplary ferrule 240 is generally cylindrical in shape and includes a first portion 292 and a second portion 294 with a transition 296 therebetween. The inner diameter of the first portion 292 is greater than the inner diameter of the second portion 294 and, in the illustrated embodiment, the outer diameter is the same. The inner diameter of the first portion 292 is also slightly greater than the outer diameter of the crimp body support 284, thereby defining a thin gap 298 therebetween for the coaxial cable metallic shield 212. The exterior surface of the second portion 294 may include an orientation groove 300. The orientation groove 300 distinguishes the second portion 294 from the first portion 292 and provides a guide as to the manner in which the ferrule 240 should be oriented during assembly. Suitable materials for the ferrule 240 include, but are not limited to, aluminum, brass and Copper.

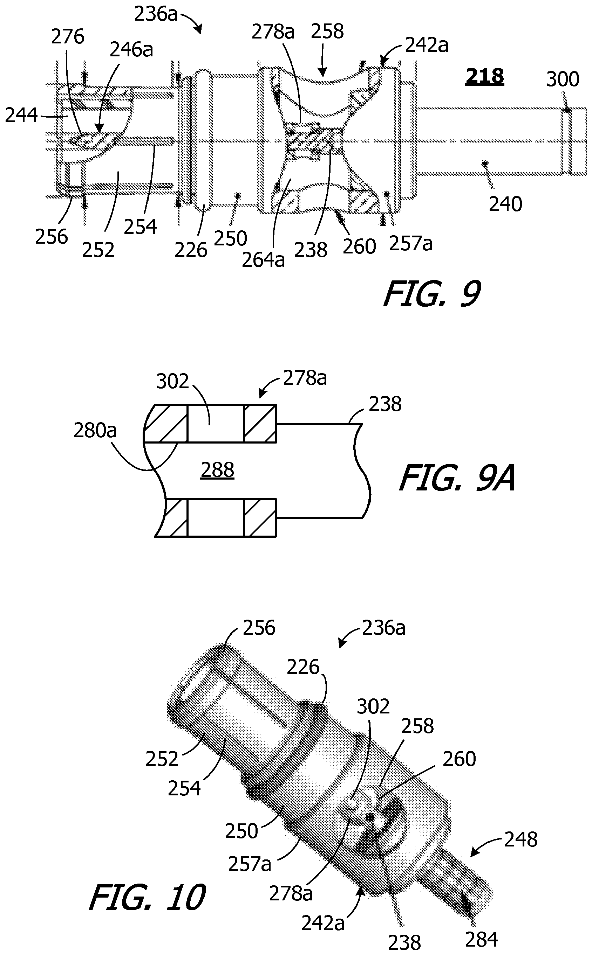

Turning to FIGS. 9 and 10, the exemplary connector assembly 218 is substantially similar to the connector assembly 216 illustrated in FIGS. 6-8 and similar elements are represented by similar reference numerals. Here, however, the configuration of the connector assembly 218 is such that the connector 206 has an overall linear shape (FIGS. 4 and 5) and is coaxial with the cable 202.

The exemplary connector assembly 218 includes a plug 236a, a crimp pin 238 and a ferrule 240. The crimp pin 238 and ferrule 240 are identical to those described above with reference to FIGS. 6-8. Here too, during assembly, the crimp pin 238 is crimped to the center conductor 208 of the coaxial cable 202 (FIG. 3) prior to the center conductor and crimp pin being inserted into the plug 236a. The crimp pin 238 is soldered to the plug 236a during the assembly process and the ferrule 240 is crimped over the coaxial cable 202 in two longitudinally spaced locations.

With respect to the specifics of the exemplary plug 236a, the plug has an outer conductor 242a, an insulator 244, a center conductor 246a, and a crimp body 248. The outer conductor 242a includes a base 250, an annular barrel 252 with gaps 254 and protrusions 256, and a hub 257a. The gaps 254 allow the barrel 252 to flex inwardly when the plug 236a is inserted into, and the protrusions engage the inner surface of, the corresponding connector. Apertures 258, 260 and 262 (see FIG. 6) extend through the hub 257 and into an open interior region 264a and are used for the purposes discussed above. The center conductor 246a is identical to center conductor 246 but for the configuration of the seat for the crimp pin 238. Referring to FIG. 9A, the seat 278a has a lumen 280a for the crimp pin 238 and solder apertures 302. The lumen 280a is coaxial with the center conductor post 276 and, accordingly, so is the crimp pin 238 and cable 202 (FIG. 5) after assembly. Suitable materials for the outer conductor 242a and center conductor 246a include, but are not limited to, those discussed above with reference to the outer conductor 242 and center conductor 246. The exemplary connector plug 236a also includes seals (not shown) that are compressed between the insulator 244 and the center conductor 246 as well as between the insulator and the outer conductor 242a in the manner illustrated in FIG. 6.

The connectors 204 and 206 described above are especially useful in to context of interconnects that require relatively small connectors. To that end, in one exemplary relatively small implementation of the connector 204, the overall length of the outer conductor 242 is about 11.4 mm (0.45 inches), the outer diameter of the outer conductor base 250 is about 4.06 mm (0.16 inches), the outer diameter of the outer conductor barrel 252 is about 3.3 mm (0.13 inches), and the width of the outer conductor base 257 (measured horizontally in FIG. 7) is about 4.57 mm (0.18 inches). The diameter of the aperture 258 is about 3.05 mm (0.12 inches), while the diameter of the aperture 260 is about 1.52 mm (0.06 inches). The outer diameter of the crimp body 248 is about 2.03 mm (0.08 inches). The overall length of the crimp pin 238 is about 3.56 mm (0.14 inches), with the diameter of the main portion 286 about 0.76 mm (0.03 inches), the diameter of the narrow portion 288 about 0.51 mm (0.02 inches) and the diameter of the lumen 290 is about 0.51 mm (0.02 inches). The length of the ferrule 248 is about 5.33 mm (0.21 inches), with the inner diameter of both the first and second portions 292 and 294 about 2.03 mm (0.08 inches). In one exemplary relatively small implementation of the connector 206, the overall length of the outer conductor 242a is about 11.2 mm (0.44 inches), the diameter of the aperture 258 is about 2,79 mm (0.11 inches), while the diameter of the aperture 260 is about 1.52 mm (0.06 inches). The dimensions of the other elements are the same as those in connector 204. Other exemplary relatively small connectors include those with dimensions that are about +/- 20% of those discussed above, as well as those connectors having any and all dimensions therebetween.

In the illustrated implementation, one of the connectors (i.e., connector 204) has an overall L-shape and the other connector (i.e., connector 206) has an overall linear shape. In other implementations, both of the connectors may have an overall L-shape or both of the connectors may have an overall linear shape. Additionally, in those instances where one or both of the connectors is non-linear, the angles may be the same or different, and may be angles other than 90 degrees.

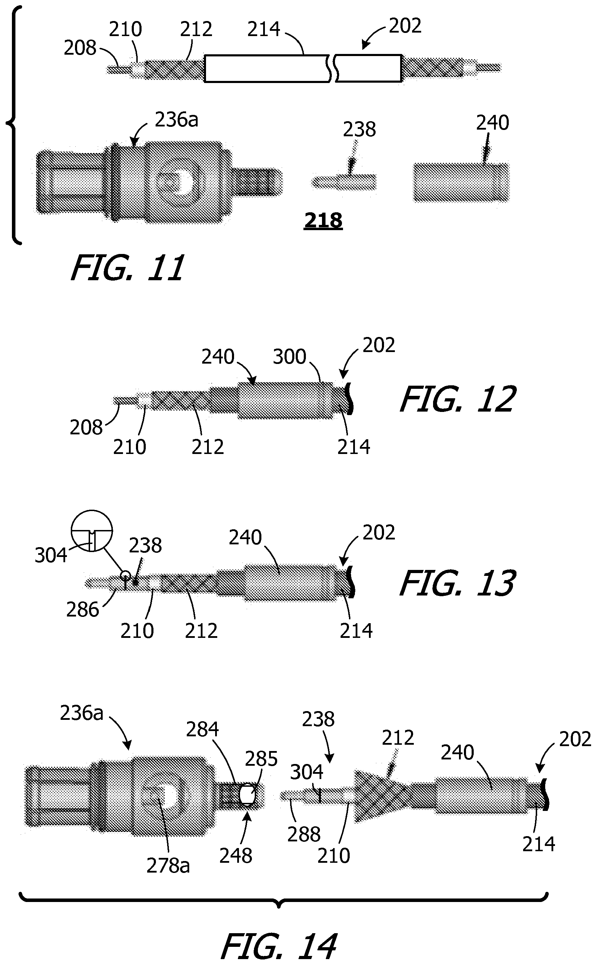

An exemplary method of assembling the interconnect 200 will now be described with reference to FIG. 11-18. The method involves attaching the connector assemblies 216 and 218 to the coaxial cable 202 and then molding the outer non-conductive portions 220 and 222 onto the attached connector assemblies and adjacent portions of the cable to complete the first and second connectors 204 and 206. Although the method steps are described in the context of one of the connectors in the interest of brevity, i.e., connector 206, identical steps are employed to assemble the connector 204. Additionally, it should be noted that unless otherwise indicated, the order of many of the steps (e.g., the steps illustrated in FIGS. 12 and 13) may be reversed.

Referring first to FIG. 11, the coaxial cable 202 is prepared for the assembly process by removing portions of the dielectric insulator 210, metallic shield 212, and jacket 214 in the manner shown. The connector assembly 218, which includes the plug 236a, crimp pin 238 and ferrule 240, is provided in a disassembled state.

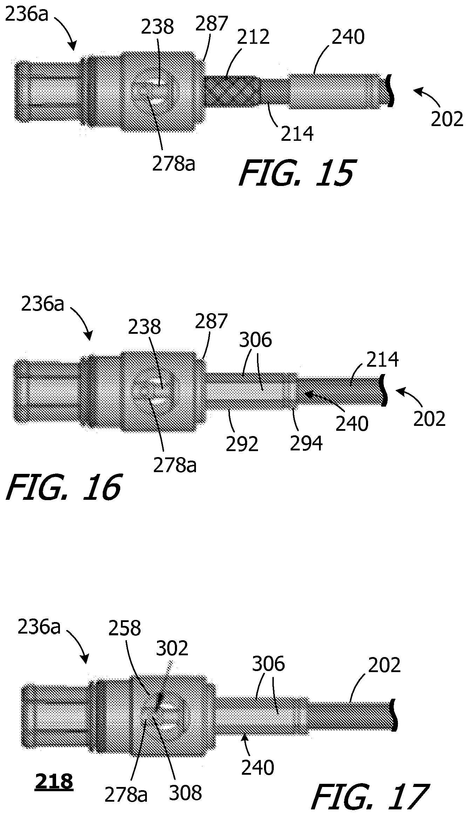

The ferrule 240 is positioned on the coaxial cable 202 and, as illustrated in FIG. 12, oriented such that the end of the ferrule without the orientation groove 300 is adjacent to end of the jacket 214. Such orientation insures that the ferrule first and second portions 292 and 294 will be respectively aligned with the exposed portion of the metallic shield 212 and the jacket 214 when the ferrule 240 is crimped onto the cable 202 and plug 236a in the manner described below with reference to FIG. 16.

The exposed end of the coaxial cable center conductor 208 is then inserted into the crimp pin lumen 290, and the crimp pin 238 is crimped to the center conductor (FIG. 13) with a suitable die. The crimp 304 may be a circular indentation in the crimp pin main portion 286 that extends into the center conductor 208 (as shown) or any other suitable deformation that interconnects the center conductor and crimp pin 238.

Turning to FIG. 14, the exposed portion of the metallic shield 212 is spread apart so that it can be positioned over the support 284 of the crimp body 248. The crimp pin 238 is then aligned with the crimp body lumen 285. The crimp pin 238 passes through the crimp body lumen 285, until the crimp pin narrow portion 288 is located in the seat 278a, as the plug 236a is positioned over the crimp pin 238 and exposed insulator 210. The metallic shield 212 is then compressed onto the crimp body support 284, as shown in FIG. 15.

Next, the ferrule 240 is moved into contact with the crimp body abutment 287. Given the respective dimensions of the ferrule 240 and the crimp body support 284, the ferrule first portion 292 is positioned over the metallic shield 212 and crimp body 248, and the ferrule second portion 294 is positioned over the jacket 214. The ferrule 240 may then crimped to the crimp body 248, with the metallic shield 212 in between, and also crimped to an intact portion of the coaxial cable 202 (i.e., a portion whose cross-section includes the center conductor 208, insulator 210, metallic shield 212 and jacket 214). A variety of crimp techniques may be employed. For example two spaced crimps similar to crimp 304 may be formed in the ferrule first and second portions 292 and 294. In the illustrated implementation, a hex die is used to crimp the entire ferrule 240, from one longitudinal end of the ferrule to the other, to the crimp body 248 and intact portion of the coaxial cable 202. Such a crimp forms six longitudinally extending deformations 306 in the ferrule 240, the crimp body 248, and the jacket 214.

The crimp pin 238 may then be soldered to the seat 278a on the connector assembly plug 236a. In particular, and referring to FIG. 17, a soldering iron (not shown) may be inserted into the plug 236a and solder 308, e.g., soft solder, may be applied to both the crimp pin 238 and the seat 278a by way of one of the solder apertures 302. As such, the solder 308 secures the crimp pin 238 to the plug 236a, as opposed to the conventional method of directly soldering a cable center conductor to a plug.

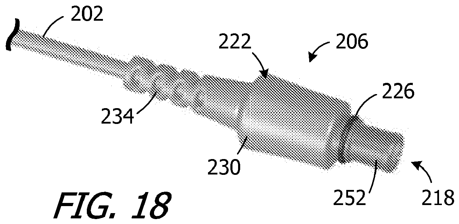

Next, the outer non-conductive portion 222 (FIG. 18) may be formed over the end portion of the cable 202 and, with the exception of the seal 226 and barrel 252, the connector assembly 218. For example, an insert molding process may be used to mold a suitable electrically non-conductive material (e.g., Riteflex® polyester elastomer, Xylex polycarbonate/amorphous polyester blend or Santoprene® thermoplastic elastomer) over the appropriate portions of the cable 202 and connector assembly 218.

Although the inventions disclosed herein have been described in terms of the preferred embodiments above, numerous modifications and/or additions to the above-described preferred embodiments would be readily apparent to one skilled in the art.

By way of example, but not limitation, the inventions include any combination of the elements from the various species and embodiments disclosed in the specification that are not already described. The inventions also include interconnects where one end is permanently connected to the associated headpiece and the other end includes one of the connectors 204 and 206. Additionally, the inventions described herein are also applicable to interconnects that are used with BTE sound processors. The present inventions also include ICS systems that include a sound processor (e.g., a body worn or BTE sound processor), a cochlear implant, a headpiece, and an interconnect as described above and/or claimed below.

## Claims

1. An interconnect for use with a headpiece, comprising:
a coaxial cable (202) including a center conductor (208) and an outer shield (212);
a plug (236) including a center conductor (246) and an outer conductor (242);
and a crimp pin (238) crimped to the cable center conductor and secured to the plug center conductor (208),
**characterized in that** the crimp pin (238) is soldered (239) directly to the plug center conductor (208).

2. An interconnect as claimed in claim 1, wherein the plug center conductor (246) includes a seat (278) and a portion of the crimp pin (238) is located within the seat.

3. An interconnect as claimed in claim 2, wherein
the plug (236) includes an open interior region (264) and an aperture (258) adjacent to the interior region; and
the seat (278) is located within the open interior region.

4. An interconnect as claimed in claim 3, wherein
the aperture (258) is about 3.05 mm (0.12 inches) in diameter.

5. An interconnect as claimed in claim 1, wherein
the plug (236) includes a crimp body (248); and
a portion of the cable outer shield (212) is located over the crimp body.

6. An interconnect as claimed in claim 5, further comprising:
a ferrule (240) that is crimped to the crimp body (248), with the portion of the cable outer shield (212) between the crimp body and ferrule, and is crimped to an intact portion of the coaxial cable (202).

7. An interconnect as claimed in claim 6, further comprising:
an electrically non-conductive member (220) over a portion of the plug (236) and over the ferrule (240).

8. An interconnect as claimed in claim 1, wherein the plug (236) defines a first plug and the crimp pin (238) defines a first crimp pin, the interconnect further comprising:
a second plug (236a) including a center conductor (246a) and an outer conductor (242a); and
a second crimp pin (238) crimped to the cable center conductor (208) and secured to the second plug center conductor.

9. An interconnect as claimed in claim 1, wherein
the plug includes a crimp body (248) connected to the outer conductor (242);
the coaxial cable outer shield (212) has a portion positioned over the crimp body; and the interconnect further comprises
a ferrule (240) crimped to the crimp body, with the portion of the cable outer shield between the crimp body and ferrule, and crimped to an intact portion of the coaxial cable (202).

10. An interconnect as claimed in claim 9, wherein the crimp body (248) comprises an annular member (248) with a knurled outer surface.

11. An interconnect as claimed in claim 9, wherein
the ferrule (240) includes a first portion (292) with a first inner diameter and a second portion (294) with a second inner diameter that is less than the first inner diameter;
the first portion of the ferrule is crimped to the crimp body (248); and
the second portion of the ferrule is crimped to the intact portion of the coaxial cable (202).

12. A headpiece assembly, comprising:
a headpiece (102) configured to communicate with a cochlear implant (104); and
an interconnect as claimed in claims 1-11 operably connected to the headpiece.

13. A method of connecting a plug (236) to a coaxial cable (202), comprising the steps of:
crimping a crimp pin (238) to a center conductor (246) of the coaxial cable; and
**characterized in that** the method further comprises soldering the crimp pin directly to the plug.

14. A method as claimed in claim 13, wherein the step of crimping a crimp pin comprises:
inserting a center conductor (208) of a coaxial cable (202) into a portion of a crimp pin (238) while the crimp pin is outside the plug (236); and
crimping the portion of the crimp pin into which the center conductor has been inserted.

15. A method as claimed in claim 13, wherein the step of soldering the crimp pin comprises:
soldering the crimp pin (238) directly to a the plug (236) with a soldering iron that has been inserted into the plug.

## Patentansprüche

1. Verbindung zur Verwendung mit einem Kopfstück, mit:
einem Koaxialkabel (202) mit einem zentralen Leiter (208) und einer äußeren Abschirmung (212);
einem Stopfen (236) einschließlich eines zentralen Leiters (246) und eines äußeren Leiters (242); und
einem Crimpstift (238), der mit dem zentralen Leiter des Kabels gecrimpt ist und an dem zentralen Leiter (208) des Stopfens befestigt ist,
**dadurch gekennzeichnet, dass** der Crimpstift (238) direkt mit dem zentralen Leiter (208) des Stopfens verlötet (239) ist.

2. Verbindung gemäß Anspruch 1, wobei der zentrale Leiter (246) des Stopfens einen Sitz (278) beinhaltet und ein Abschnitt des Crimpstifts (238) innerhalb des Sitzes angeordnet ist.

3. Verbindung gemäß Anspruch 2, wobei
der Stopfen (236) einen offenen Innenbereich (264) und eine Öffnung (258) benachbart zu dem Innenbereich aufweist; und
der Sitz (278) innerhalb des offenen Innenbereichs angeordnet ist.

4. Verbindung gemäß Anspruch 3, wobei die Öffnung (258) einen Durchmesser von etwa 3,05 mm (0,12 inch) aufweist.

5. Verbindung gemäß Anspruch 1, wobei der Stopfen (236) einen Crimpkörper (248) aufweist und ein Abschnitt der äußeren Abschirmung (212) des Kabels über dem Crimpkörper angeordnet ist.

6. Verbindung gemäß Anspruch 5, ferner versehen mit: einer Hülse (240), die auf den Crimpkörper (248) gecrimpt ist, mit dem Abschnitt der äußeren Abschirmung (212) des Kabels zwischen dem Crimpkörper und der Hülse, und die auf einen intakten Abschnitt des Koaxialkabels (202) gecrimpt ist.

7. Verbindung gemäß Anspruch 6, ferner versehen mit: einem elektrisch nichtleitenden Bauteil (220) über einem Abschnitt des Stopfens (236) und über der Hülse (240).

8. Verbindung gemäß Anspruch 1, wobei der Stopfen (236) einen ersten Stopfen festlegt und der Crimpstift (238) einen ersten Crimpstift festlegt, und wobei die Verbindung ferner versehen ist mit:
einem zweiten Stopfen (236a) mit einem zentralen Leiter (246a) und einem äußeren Leiter (242a); und
einem zweiten Crimpstift (238), der auf den zentralen Leiter (208) des Kabels gecrimpt ist und an dem zentralen Leiter des zweiten Stopfens befestigt ist.

9. Verbindung gemäß Anspruch 1, wobei
der Stopfen einen Crimpkörper (248) beinhaltet, der mit dem äußeren Leiter (242) verbunden ist;
die äußere Abschirmung (212) des Koaxialkabels einen Abschnitt aufweist, der über dem Crimpkörper angeordnet ist; und die Verbindung ferner versehen ist mit:
einer Hülse (240), die auf den Crimpkörper gecrimpt ist, mit dem Abschnitt der äußeren Abschirmung des Kabels zwischen dem Crimpkörper und der Hülse, und die auf einen intakten Abschnitt des Koaxialkabels (202) gecrimpt ist.

10. Verbindung gemäß Anspruch 9, wobei der Crimpkörper (248) ein ringförmiges Bauteil (248) mit einer geriffelten äußeren Oberfläche aufweist.

11. Verbindung gemäß Anspruch 9, wobei
die Hülse (240) einen ersten Abschnitt (292) mit einem ersten Innendurchmesser und einem zweiten Abschnitt (294) mit einem zweiten Innendurchmesser kleiner als der erste Innendurchmesser aufweist;
der erste Abschnitt der Hülse auf den Crimpkörper (248) gecrimpt ist; und
der zweite Abschnitt der Hülse auf den intakten Bereich des Koaxialkabels (202) gecrimpt ist.

12. Kopfstückbaugruppe mit:
einem Kopfstück (102) zum Kommunizieren mit einem Cochlearimplantat (104); und
einer Verbindung gemäß einem der Ansprüche 1-11, die in Wirkverbindung mit dem Kopfstück steht.

13. Verfahren zum Verbinden eines Stopfens (236) mit einem Koaxialkabel (202), wobei:
ein Crimpstift (238) auf einen zentralen Leiter (246) des Koaxialkabels gecrimpt wird; und
**dadurch gekennzeichnet, dass** ferner der Crimpstift direkt mit dem Stopfen verlötet wird.

14. Verfahren gemäß Anspruch 13, wobei beim Crimpen eines Crimpstifts:
ein zentraler Leiter (208) eines Koaxialkabels (202) in einen Abschnitt eines Crimpstifts (238) eingeführt wird, während der Crimpstift außerhalb des Stopfens (236) ist; und
der Abschnitt des Crimpstifts, in welchen der zentrale Leiter eingeführt wurde, gecrimpt wird.

15. Verfahren gemäß Anspruch 13, wobei beim Schritt des Lötens des Crimpstifts der Crimpstift (238) mit einem Löteisen, weiches in den Stopfen eingeführt wurde, direkt mit dem Stopfen (236) verlötet wird.

## Revendications

1. Interconnexion à utiliser avec un casque, comprenant :
un câble coaxial (202) comprenant un conducteur central (208) et une protection extérieure (212) ; et
une fiche (236) comprenant un conducteur central (246) et un conducteur extérieur (242) ; et
une broche sertie (238) sertie sur le conducteur central de câble et fixée de façon sécurisée au conducteur central de fiche (208) ;
**caractérisée en ce que** la broche sertie (238) est soudée (239) directement sur le conducteur central de fiche (208).

2. Interconnexion selon la revendication 1, dans laquelle le conducteur central de fiche (246) comprend un siège (278) et dans laquelle une partie de la broche sertie (238) est positionnée à l'intérieur du siège.

3. Interconnexion selon la revendication 2, dans laquelle:
la fiche (236) comprend une région intérieure ouverte (264) et une ouverture (258) adjacente à la région intérieure ; et
le siège (278) est positionné à l'intérieur de la région intérieure ouverte.

4. Interconnexion selon la revendication 3, dans laquelle l'ouverture (258) a un diamètre d'environ 3,05 mm (0,12 pouce).

5. Interconnexion selon la revendication 1, dans laquelle:
la fiche (236) comprend un corps serti (248) ; et
une partie de la protection extérieure de câble (212) est positionnée au-dessus du corps serti.

6. Interconnexion selon la revendication 5, comprenant en outre:
une férule (240) sertie sur le corps serti (248), avec la partie de la protection extérieure de câble (212) située entre le corps serti et la férule, et sertie sur une partie intacte du câble coaxial (202).

7. Interconnexion selon la revendication 6, comprenant en outre:
un élément électriquement non conducteur (220) situé au-dessus d'une partie de la fiche (236) et au-dessus de la férule (240).

8. Interconnexion selon la revendication 1, dans laquelle la fiche (236) définit une première fiche et dans laquelle la broche sertie (238) définit une première broche sertie, l'interconnexion comprenant en outre:
une seconde fiche (236a) comprenant un conducteur central (246a) et un conducteur extérieur (242a) ; et
une seconde broche sertie (238) sertie sur le conducteur central de câble (208) et fixée de façon sécurisée au second conducteur central de fiche.

9. Interconnexion selon la revendication 1, dans laquelle:
la fiche comprend un corps serti (248) connecté au conducteur extérieur (242) ;
la protection extérieure de câble coaxial (212) a une partie positionnée au-dessus du corps serti; et
l'interconnexion comprend en outre:
une férule (240) sertie sur le corps serti, avec la partie de la protection extérieure de câble prévue entre le corps serti et la férule, et sertie sur une partie intacte du câble coaxial (202).

10. Interconnexion selon la revendication 9, dans laquelle le corps serti (248) comprend un élément annulaire (248) avec une surface extérieure moletée.

11. Interconnexion selon la revendication 9, dans laquelle:
la férule (240) comprend une première partie (292) avec un premier diamètre intérieur et une seconde partie (294) avec un second diamètre intérieur inférieur au premier diamètre intérieur ;
la première partie de la férule est sertie sur le corps serti (248) ; et
la seconde partie de la férule est sertie sur la partie intacte du câble coaxial (202).

12. Ensemble casque, comprenant:
un casque (102) configuré pour communiquer avec un implant cochléaire (104) ; et
une interconnexion selon les revendications 1 à 11 connectée en fonctionnement au casque.

13. Procédé de connexion d'une fiche (236) à un câble coaxial (202), comprenant les étapes consistant à:
sertir une broche sertie (238) sur un conducteur central (246) du câble coaxial ; et
**caractérisé en ce que** le procédé comprend en outre la soudure de la broche sertie directement sur la fiche.

14. Procédé selon la revendication 13, dans lequel l'étape de sertissage d'une broche sertie comprend:
l'insertion d'un conducteur central (208) d'un câble coaxial (202) à l'intérieur d'une partie d'une broche sertie (238) tandis que la broche sertie est placée à l'extérieur de la fiche (236) ; et
le sertissage de la partie de la broche sertie dans laquelle le conducteur central a été inséré.

15. Procédé selon la revendication 13, dans lequel l'étape de soudure de la broche sertie comprend:
la soudure de la broche sertie (238) directement sur la fiche (236) avec un fer de soudure inséré dans la fiche.
